Europäisches Patentamt

European Patent Office

Office européen des brevets

(19)

(11) Publication number: **0 055 495**
**B1**

# (12) EUROPEAN PATENT SPECIFICATION

(45) Date of publication of patent specification: **20.06.84**

(21) Application number: **81201238.3**

(22) Date of filing: **30.10.81**

(51) Int. Cl.³: **C 07 C  5/11,  C 07 C  13/20,
B 01 J  23/58 //C07C35/08**

(54) Process for the preparation of a cycloalkene through partial hydrogenation of the corresponding aromatic hydrocarbon.

(30) Priority: **31.12.80 NL 8007111
02.09.81 NL 8104067**

(43) Date of publication of application:
**07.07.82 Bulletin 82/27**

(45) Publication of the grant of the patent:
**20.06.84 Bulletin 84/25**

(84) Designated Contracting States:
**BE CH DE FR GB IT LI NL**

(56) References cited:
**GB - A - 1 094 911
GB - A - 1 381 048
US - A - 3 183 278
US - A - 3 912 787
US - A - 4 055 512**

**CHEMICAL ABSTRACTS, vol. 91, no. 5, July 30,
1979 page 592, abstract 39006k Columbus,
Ohio, US**

(73) Proprietor: **STAMICARBON B.V.
Postbus 10
NL-6160 MC  Geleen (NL)**

(72) Inventor: **Don, Johannes Albertus
De Savorin Lohmanstraat 132
NL-3904 AW Veenendaal (NL)**
Inventor: **Scholten, Joseph Johannes Franciscus
Sartonlaan 7
NL-6132 BC Sittard (NL)**

(74) Representative: **Pinckaers, August René et al,
OCTROOIBUREAU DSM Postbus 9
NL-6160 MA  Geleen (NL)**

Courier Press, Leamington Spa, England.

## Description

The invention relates to a process for the preparation of cycloalkene, in particular cyclohexene, through partial hydrogenation of the corresponding aromatic hydrocarbon, in particular benzene, in the gas phase in the presence of a ruthenium catalyst.

A similar process is known from the Dutch patent application 6403702 laid open to public inspection. According to the known process, the aromatic hydrocarbon (in the vapour phase) which is to be converted is brought into contact with metallic ruthenium, for instance ruthenium-black, together with hydrogen and 5—50% by weight (relative to the hydrocarbon) of an alcohol, at a temperature of 15—150°C. The ruthenium may be applied to a support. From an experiment it appears that it is possible to prepare cyclohexene from benzene at a conversion of 10% and a selectivity of 7%.

What has been said above reveals the great disadvantage of the known process, viz. the very low selectivity to the desired alkene, even at a low rate of conversion of the hydrocarbon.

The process according to the invention yields a relatively much higher selectivity to the desired cycloalkene. According to the invention the preparation of a cycloalkene through partial hydrogenation of the corresponding aromatic hydrocarbon in the gas phase in the presence of a ruthenium cataliyst is characterized in that this partial hydrogenation is carried out continuously in the presence of water vapour preferably with a partial pressure between 50 and 99%, in particular between 75 and 95%, of its saturation pressure under the prevailing con-, ditions, the partial pressure of the hydrocarbon to be converted ranging preferably from 1 to 99%, in particular from 10 to 95%, more in particular from 25 to 95%, of its saturation pressure under the prevailing conditions. The water may also be formed in situ from oxygen and hydrogen.

From the Dutch patent application 7205832 laid open to public inspection it is known to hydrogenate benzene to cyclohexene in the presence of water, but this concerns a liquid-phase process in which the water is used together with an alkaline substance to maintain a liquid phase with a pH above 7.5.

Liquid-phase hydrogenation, however, has some major drawbacks. Among other things, liquid-phase hydrogenation is more unsafe, is much more difficult to carry out continuously, demands more complex equipment, especially for separating the reactants from the catalyst suspension, and demands a relatively higher pressure than gas-phase hydrogenation.

The process according to the invention is preferably carried out at a temperature between 250 and 600 K, in particular between 290 and 500 K, and at a pressure between 0.01 and 10 MPa. Although higher and lower pressures are practicable they do not offer any advantage.

The ruthenium catalyst used in the process according to the invention is by preferene metallic Ru, whether or not on a support, preferably a hydrophobic support, like porous corundum, silica dehydrated at high temperature, silanized silica and mixtures of these.

Besides Ru, the ruthenium catalyst may also contain one or more different metals or compounds of metals (e.g. oxides), preferably base metals such as Fe, Cr, Ni, Ge, Pb, Zn and particularly Na. Than the catalyst contains preferably 0.1—10% by weight, relative to the ruthenenium in the catalyst, of Na. Measurements show that the presence of Na, e.g. approx. 1% by weight of $Na_2O$, causes a sharp improvement of the stability of the catalyst.

Preferably, before being used in the partial hydrogenation the ruthenium catalyst undergoes preliminary treatment with water, for instance by leading hydrogen saturated with water over this catalyst for 0.1—1 hour.

By preference, the surface of the catalyst is covered with as little halogen, notably chlorine, as possible, since the activity of the catalyst is greatly reduced by this deposit. The commonest way of preparing metallic ruthenium is the reduction, at 575—675 K, of technically pure $RuO_2$, during which process the chlorine present on the technically pure $RuO_2$ (approx. 0.4% by weight) is scarcely removed. Therefore it is to be preferred to make the technically pure $RuO_2$ substantially chlorine-free before reducing it, by treating it with air for, say, 0.1—10 hours, at a high temperature of, say, 1250—1500 K, or to start with practically chlorine-free $RuO_2$ which has been prepared by converting $RuCl_3$ with air at a temperature above 1000 K and preferably below 1400 K.

In the process according to the invention the reaction product consists of a mixture of cycloalkene, cycloalkane, water and unconverted starting materials. Because of the presence of water and cycloalkene this mixture is extremely well suited for the conversion of the cycloalkene present and the water present (adding extra water if necessary) into cycloalkanol. This hydration process may be effected in any known way.

The invention will be elucidated with reference to the following non-restricting examples. The Ru catalyst applied consisted of Ru powder made by treating technically pure $RuO_2$ with air for 1 hour at 1250—1500 K and by subsequently reducing this $RuO_2$ with hydrogen. In examples 1 and 2 the Ru powder thus prepared in addition underwent preliminary treatment with water, in that hydrogen gas saturated with water was passed over it for 10 minutes

## Example 1

At 295 K and 110 kPa per minute 30 cm³ (NTP) of a gas mixture consisting of 0.8 molar parts of water, 1 molar part of benzene and 30 molar parts of hydrogen was passed over 6.5 mg Ru powder. At a conversion of 6%, per mole of converted benzene 0.16 mole of cyclohexene was formed.

### Example 2

At 318 K and 110 kPa per minute 21 cm³ (NTP) of a gas mixture consisting of 1.3 molar parts of water, 1 molar part of benzene, 4 molar parts of hydrogen and 12 molar parts of helium was passed over 7.2 mg Ru powder. At a conversion of 49%, per mole of converted benzene 0.21 mole of cylcohexene was formed; at a conversion of 20%, per mole of converted benzene 0.42 mole of cyclohexene was formed and at a conversion of 3.4%, per mole of converted benzene 0.65 mole of cyclohexene was formed.

### Example 3

At 295 K and 110 kPa per minute 30 ml (NTP) of a gas mixture consisting of 0.8 molar parts of water, 1 molar part of benzene and 30 molar parts of hydrogen was passed over 3 mg Ru powder. At a conversion of 22%, per mole of converted benzene 0.08 mole of cyclohexene was formed.

### Claims

1. Process for the preparation of a cycloalkene through partial hydrogenation of the corresponding aromatic hydrocarbon in the gas phase in the presence of a ruthenium catalyst, characterized in that this hydrogenation is carried out continuously in the presence of water vapour.

2. Process according to claim 1, characterized in that cyclohexene is prepared by partial hydrogenation of benzene.

3. Process according to claim 1 or 2, characterized in that the partial hydrogenation is carried out at a temperature between 250 and 600 K.

4. Process according to one of the claims 1—3, characterized in that a pressure between 0.01 and 10 MPa is used.

5. Processs according to one of the claims 1—4, characterized in that the partial hydrogenation is carried out in the presence of water vapour with a partial pressure between 50 and 99% of its saturation pressure under the prevailing conditions.

6. Process according to one of the claims 1—5, characterized in that the partial hydrogenation is carried out at a partial pressure of the hydrocarbon to be converted between 1 and 99% of its saturation pressure under the prevailing conditions.

7. Process according to one of the claims 1—6, characterized in that the ruthenium catalyst contains 0.1—10% by weight, relative to the ruthenium in the catalyst, of Na.

8. Process according to one of the claims 1—7, characterized in that the ruthenium catalyst, before being used in the partial hydrogenation, undergoes preliminary treatment with water.

9. Process according to one of the claims 1—8, characterized in that the surface of the ruthenium catalyst is substantially halogen-free.

### Revendications

1. Procédé de préparation d'un cyclo-alcène par hydrogénation partielle de l'hydrocarbure aromatique correspondant en phase gazeuse et en présence d'un catalyseur de ruthénium, caractérisé en ce que l'hydrogénation est effectuée en continu en présence de vapeur d'eau.

2. Procédé selon la revendication 1, caractérisé en ce que le cyclohexène est préparé par hydrogénation partielle de benzène.

3. Procédé selon l'une des revendications 1 et 2, caractérisé en ce que l'hydrogénation partielle est effectuée à une température située entre 250 et 600 K.

4. Procédé selon l'une des revendications 1 à 3, caractérisé en ce qu'on applique un pression située entre 0,01 et 10 MPa.

5. Procédé selon l'une des revendications 1 à 4, caractérisé en ce que l'hydrogénation partielle est effectuée en présence de vapeur d'eau avec une pression partielle située entre 50 et 99% de sa pression de saturation, dans les conditions régnantes.

6. Procédé selon l'une des revendications 1 à 5, caractérisé en ce que l'hydrogénation partielle est effectuée à une pression partielle de l'hydrocarbure à convertir située entre 1 et 99% de sa pression de saturation dans les conditions régnantes.

7. Procédé selon l'une des revendications 1 à 6, caractérisé en ce que le catalyseur de ruthénium contient 0,1 à 10% en poids de Na, calculé par rapport au ruthénium se trouvant dans le catalyseur.

8. Procédé selon l'une des revendications 1 à 7, caractérisé en ce que le catalyseur de ruthénium est soumis — avant d'être utilisé dans l'hydrogénation partielle — à un traitement préalable avec de l'eau.

9. Procédé selon l'une des revendications 1 à 8, caractérisé en ce que la surface du catalyseur de ruthénium est essentiellement exempt d'halogène.

### Patentansprüche

1. Verfahren zur Herstellung eines Cycloalkens durch Partialhydrierung des korrespondierenden aromatischen Kohlenwasserstoffs in der Gasphase in Anwesenheit eines Ruthenium-Katalysators, dadurch gekennzeichnet, daß diese Hydrierung kontinuierlich in Anwesenheit von Wasserdampf durchgeführt wird.

2. Verfahren nach Anspruch 1, dadurch gekennzeichnet, daß Cyclohexen durch Partialhydrierung von Benzol hergestellt wird.

3. Verfahren nach Anspruch 1 oder 2, dadurch gekennzeichnet, daß die Partialhydrierung bei einer Temperatur zwischen 250 und 600 K durchgeführt wird.

4. Verfahren nach einem der Ansprüche 1—3, dadurch gekennzeichnet, daß ein Druck zwischen 0,01 und 10 MPa angewandt wird.

5. Verfahren nach einem der Ansprüche 1—4, dadurch gekennzeichnet, daß die Partialhydrierung in Anwesenheit von Wasserdampf mit einem Partialdruck zwischen 50 und 99% seines Sättigungsdruckes unter den vorherrschenden Bedingungen durchgeführt wird.

6. Verfahren nach einem der Ansprüche 1—5, dadurch gekennzeichnet, daß die Partialhydrierung bei einem Partialdruck des umzuwandelnden Kohlenwasserstoffs zwischen 1 und 99% seines Sättigungsdruckes unter den vorherrschenden Umständen durchgeführt wird.

7. Verfahren nach einem der Ansprüche 1—6, dadurch gekennzeichnet, daß der Ruthenium-Katalysator 0,1—10 Gew.-% Na enthält, und zwar je nach dem Ruthenium im Katalysator.

8. Verfahren nach einem der Ansprüche 1—7, dadurch gekennzeichnet, daß der Ruthenium-Katalysator vor der Verwendung in der Partialhydrierung einer Vorbehandlung mit Wasser unterzogen wird.

9. Verfahren nach einem der Ansprüche 1—8, dadurch gekennzeichnet, daß die Oberfläche des Ruthenium-Katalysators im wesentlichen halogenfrei ist.